# EUROPÄISCHE PATENTSCHRIFT

(11) **EP 4 389 219 B1**
(45) Veröffentlichungstag und Bekanntmachung des Hinweises auf die Patenterteilung: **10.09.2025**
(21) Anmeldenummer: 22215412.2
(22) Anmeldetag: 21.12.2022
(51) Int. Cl.: A61P 31/04, A01N 43/40, C07D 213/74, A01P 1/00, A01P 3/00

(54) **ANTIMIKROBIELLE VERBINDUNG, VERFAHREN ZU DEREN HERSTELLUNG UND VERWENDUNG DER ANTIMIKROBIELLEN VERBINDUNG ZUR HERSTELLUNG EINER KOSMETISCHEN ODER PHARMAZEUTISCHEN ZUSAMMENSETZUNG**
ANTIMICROBIAL COMPOUND, METHOD FOR ITS PREPARATION AND USE OF THE ANTIMICROBIAL COMPOUND FOR THE PREPARATION OF A COSMETIC OR PHARMACEUTICAL COMPOSITION
COMPOSÉ ANTIMICROBIEN, SON PROCÉDÉ DE PRÉPARATION ET UTILISATION DU COMPOSÉ ANTIMICROBIEN POUR LA PRÉPARATION D'UNE COMPOSITION COSMÉTIQUE OU PHARMACEUTIQUE

(43) Veröffentlichungstag der Anmeldung: 26.06.2024
(73) Patentinhaber: Aerochemica Dr. Deppe GmbH, 47906 Kempen (DE)
(72) Erfinder: BRUDER, Gregor, 47906 Kempen (DE); DEPPE, Marc, 47906 Kempen (DE)
(74) Vertreter: mepat Patentanwälte

(56) Entgegenhaltungen:
- EP-A2- 0 142 835
- US-A- 4 206 215
- THOMAS M ET AL: "Polycations. 17. Synthesis and properties of polycationic derivatives of carbohydrates", CARBOHYDRATE RESEARCH, PERGAMON, GB, vol. 344, no. 13, 8 September 2009 (2009-09-08), pages 1620 - 1627, XP026502115, ISSN: 0008-6215, [retrieved on 20090424], DOI: 10.1016/J.CARRES.2009.04.021
- YAKUSHCHENKO I K ET AL: "Eff ect of the linker nature on the antibacterial activity of structural analogs of octenidine", RUSSIAN CHEMICAL BULLETIN SERIYA KHIMICHESKAYA, 1 March 2022 (2022-03-01), pages 595 - 597, XP093037054, Retrieved from the Internet <URL:https://link.springer.com/article/10.1007/s11172-022-3456-8> [retrieved on 20230403]
- TROUSIL SEBASTIAN ET AL: "Design of symmetrical and nonsymmetrical N,N-dimethylaminopyridine derivatives as highly potent choline kinase alpha inhibitors", MEDCHEMCOMM, vol. 4, no. 4, 1 January 2013 (2013-01-01), United Kingdom, pages 693, XP093037042, ISSN: 2040-2503, DOI: 10.1039/c3md00068k

## Beschreibung

Die Erfindung betrifft eine antimikrobielle Verbindung, ein Verfahren zur Herstellung der antimikrobiellen Verbindung und die Verwendung der antimikrobiellen Verbindung zur Herstellung einer kosmetischen oder pharmazeutischen Zusammensetzung zur Anwendung auf Haut, sowie eine kosmetische bzw. pharmazeutische Zusammensetzung mit der antimikrobiellen Verbindung geeignet zur Anwendung auf geschädigter und ungeschädigter Haut und Schleimhaut.

Aus dem Stand der Technik ist die antimikrobielle Verbindung Octenidindihydrochlorid (*N,N'-(*Decan-1,10-diyldi-1(4*H*)-pyridyl-4-yliden)bis(octylammonium)dichlorid) als Antiseptikum mit bakteriziden, fungiziden und antiviralen Eigenschaften bekannt, die vor allem in Haut-, Schleimhaut- und Wundantiseptika eingesetzt wird. Octenidindihydrochlorid wird üblicherweise in einem Massenanteil von 0,1 bis 2,0 Gew.-% zur Desinfektion von Haut- und Schleimhautwunden sowie zur Desinfektion vor medizinischen Eingriffen verwendet und wird in unterschiedlichen Verabreichungsformen unter dem Handelsnamen Octenisept^{®} von der Schülke & Mayr GmbH, Deutschland, vertrieben.

Octenidin bzw. Octenidindihydrochlorid und dessen Derivate werden in US 4,206,215 A als antimikrobiell wirksame Verbindung Bis-[4-(R-amino)-1-pyridium]-alkan der Formel 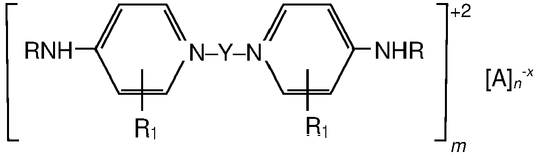 beschrieben, wobei Y eine Alkylengruppe mit 4 bis 18 Kohlenstoffatomen ist. R kann eine Alkylgruppe mit 6 bis 18 Kohlenstoffatomen, eine Cycloalkylgruppe mit 5 bis 7 Kohlenstoffatomen oder eine Phenylgruppe sein, die mit einer Metyhendioxygruppe oder einem oder zwei Substituenten aus der Gruppe substituiert ist, die Halogenatome, Niederakyl-, Niederalkyoxy-, Nitro-, Cyano- und Trifluormethylgruppen umfasst. R₁ kann Wasserstoff oder eine Alkylgruppe mit 1 bis 4 Kohlenstoffatomen sein. Das Bis-[4-(R-amino)-1-pyridium]-alkan wird durch Reaktion eines 4-(R-amino)pyridins und eines disubstituierten Alkans im molaren Verhältnis zwei zu eins erhalten. Die Reaktion erfolgt entweder in einem inerten Lösungsmittel bei 80 bis 150 °C für 15 bis 20 Stunden, oder ohne Lösungsmittel bei 120 bis 150 °C für 2 bis 6 Stunden. Das Reaktionsprodukt kann beispielsweise durch Filtration isoliert werden, wenn das Produkt im Reaktionsmedium unlöslich ist, oder durch Verdünnung der Reaktionsmischung mit einem unpolaren Lösungsmittel, um das Produkt auszufällen, oder durch Verdampfen des Reaktionsmediums, um das Produkt als Rückstand zu erhalten.

Weitere Octenidin-Derivate und ihre antimikrobielle Aktivität werden in Thomas M. et al., Carbohydrate Research, 344 (13), 2009, Seiten 1620-1627 und in Yakushchenko I. K. et al., Russian Chemical Bulletin Seriya Khimicheskaya, 2022, Seiten 595-597 beschrieben. Ausgehend von diesem Stand der Technik ist es Aufgabe der vorliegenden Erfindung, eine zum Einsatz in kosmetischen oder pharmazeutische Zusammensetzungen geeignete antimikrobielle Verbindung mit verbesserter Effizienz und Wirksamkeit bereitzustellen. Diese Aufgabe wird durch eine antimikrobielle Verbindung mit den Merkmalen des unabhängigen Anspruchs 1 gelöst.

Die weitere Aufgabe, die antimikrobielle Verbindung effizient mit hoher Ausbeute herzustellen, wird durch das Verfahren mit den Merkmalen des unabhängigen Anspruchs 7 gelöst.

Die Verwendung der antimikrobiellen Verbindung zur Herstellung einer kosmetischen oder pharmazeutischen Zusammensetzung wird durch die Merkmale des unabhängigen Anspruchs 10 offenbart.

Die weitere Aufgabe, eine verbesserte kosmetische oder pharmazeutische Zusammensetzung bereitzustellen, wird durch die kosmetische oder pharmazeutische Zusammensetzung mit den Merkmalen des unabhängigen Anspruchs 11 gelöst.

Weiterbildungen bzw. bevorzugte Ausführungsformen sind in den jeweiligen Unteransprüchen ausgeführt.

Gemäß einer ersten Ausführungsform weist die erfindungsgemäße antimikrobielle Verbindung die Formel (I) 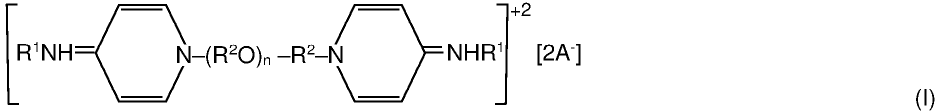 auf, wobei
R¹ ein Alkylrest mit 4 bis 18 Kohlenstoffatomen oder ein Cycloalkylrest mit 5 bis 12 Kohlenstoffatomen oder ein (Poly)alkylenglykolrest mit 1 bis 6 Alkoxyeinheiten oder ein Phenylrest oder ein Benzylrest ist, und
R² eine Alkylgruppe mit 2 oder 3 oder 4 Kohlenstoffatomen ist, und
n eine ganze Zahl von 1 bis 15 ist, und
[2A⁻] für zwei einwertige Anionen (2 A⁻) oder ein zweiwertiges Anion (A²⁻) steht.

D. h., dass die beiden 4-(R¹-amino)-pyridin-Reste der erfindungsgemäßen antimikrobiellen Verbindung durch eine Alkylenglykolkette voneinander beabstandet sind, die eine bis fünfzehn Alkoxygruppen -R²O- aufweist. Bevorzugt kann die Alkylenglykolkette zwei bis zwölf Alkoxygruppen -R²O- aufweisen. Die Alkylenglykolkette kann dabei als Alkoxygruppen Ethoxygruppen -C₂H₄O- oder n- oder iso-Propoxygruppen -C₃H₆O- oder n- oder iso-Butoxygruppen -C₄H₈O- aufweisen. Oder die Alkylenglykolkette kann zwei unterschiedliche Alkoxygruppen, Ethoxy- und Propoxy- oder Ethoxy- und Butoxy- oder Propoxy- und Butoxygruppen, oder alle drei Alkoxygruppen, Ethoxy- und Propoxy- und Butoxygruppen, in zufälliger oder abwechselnder oder blockweiser Reihenfolge, aufweisen. Alkoxygruppen werden auch als Alkylenoxideinheiten bzw. Ethoxygruppen als Ethylenoxideinheiten, Propoxygruppen als Propylenoxideinheiten und Butoxygruppen als Butylenoxideinheiten bezeichnet.

Als R¹ kann der Alkyl- oder Cycloalkylrest jeweils gesättigt oder ungesättigt, gerade oder verzweigt, einfach oder mehrfach substituiert oder unsubstituiert sein. Die Alkoxyeinheit des Alkylenglykolrests als R¹ kann Ethylenoxid- oder Propylenoxid- oder Butylenoxideinheit sein. Ist R¹ ein Polyalkylenglykolrest mit 2 bis 6 Alkoxyeinheiten, können diese Ethylenoxid- oder Propylenoxid- oder Butylenoxideinheiten oder Kombinationen daraus sein. Ein Phenyl- oder Benzylrest als R¹ kann einfach oder mehrfach substituiert oder unsubstituiert sein.

Die erfindungsgemäße antimikrobielle Verbindung weist im Vergleich zu Octenidin bzw. Octenidindihydrochlorid und dessen Derivaten eine sehr gute Wasserlöslichkeit und schon in niedriger Konzentration eine hohe Wirksamkeit auf.

Ferner ist die erfindungsgemäße antimikrobielle Verbindung gemäß einer weiteren Ausführungsform effizient und mit hoher Ausbeute aus der Reaktion eines 4-(R¹-amino)-pyridins der Formel (II) 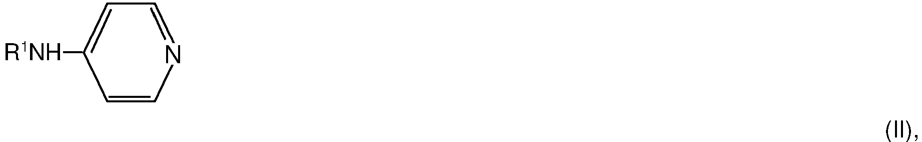 mit einem disubstituierten Polyalkylenglykolderivat der Formel (III)

X-(R²O)ₙ-R²-X (III)

in einem molaren Verhältnis von 2:1 erhaltbar. Auch hierbei ist
R¹ ein Alkylrest mit 4 bis 18 Kohlenstoffatomen oder ein Cycloalkylrest mit 5 bis 12 Kohlenstoffatomen oder ein (Poly)alkylenglykolrest mit 1 bis 6 Alkoxyeinheiten oder ein Phenylrest oder ein Benzylrest, und
R² eine Alkylgruppe mit 2 oder 3 oder 4 Kohlenstoffatomen, und
n eine ganze Zahl von 1 bis 15 ist.
X ist ein einwertiger anionischer Rest ist, wobei entweder die zwei einwertigen anionischen Reste X des disubstituierten Polyalkylenglykolderivats der Formel (III) den zwei einwertigen Anionen (2 A⁻) der antimikrobiellen Verbindung der Formel (I) entsprechen, oder nach erfolgter Reaktion des 4-(R¹-amino)-pyridins der Formel (II) mit dem disubstituierten Polyalkylenglykolderivat der Formel (III) die zwei einwertigen anionischen Reste X in einem lonentauschverfahren durch die zwei einwertigen Anionen (2 A⁻) oder das zweiwertige Anion (A²⁻) der antimikrobiellen Verbindung der Formel (I) ersetzt werden.

Nach einer weiteren Ausführungsform der erfindungsgemäßen antimikrobiellen Verbindung ist vorgesehen, dass die zwei einwertigen Anionen (2 A⁻) aus einer Gruppe ausgewählt sind, die Chlorid, Bromid, Fluorid, Iodid, Nitrat und Anionen organischer Monocarbonsäuren, wie z. B. Formiat, Acetat, Propionat, Benzoat, Nicotinat etc. aufweist, wobei Chlorid und Bromid bevorzugt sind.

Nach einer dazu alternativen Ausführungsform der erfindungsgemäßen antimikrobiellen Verbindung ist vorgesehen, dass das zweiwertige Anion (A²⁻) aus einer Gruppe ausgewählt ist, die Sulfat und Anionen organischer Dicarbonsäuren, wie z. B. Oxalat, Malonat, Adipat, Tartrat, Succinat, Phthalat etc. aufweist.

Der einwertige anionische Rest X des disubstituierten Polyalkylenglykolderivats der Formel (III) kann gemäß einer weiteren Ausführungsform der erfindungsgemäßen antimikrobiellen Verbindung aus einer Gruppe ausgewählt sein, die Chlorid, Bromid, Iodid, Alkyl- und Aryl-Sulfonyloxy-Reste sowie Metho- und Ethosulfate aufweist.

Nach einer weiteren Ausführungsform der erfindungsgemäßen antimikrobiellen Verbindung ist in der Formel (I) R¹ ein linearer Octylrest, R² eine Ethylgruppe, n gleich 3, und die zwei einwertigen Anionen (2 A⁻) sind Chlorid. Diese antimikrobielle Verbindung, die aus der Reaktion von 4-(Octylamino)-pyridin mit Tetraethylenglykoldichlorid (anderer Name Bis-[2-(2-chlorethoxy)-ethyl]-ether) im molaren Verhältnis 2:1 erhaltbar ist, kann als N,N'-(3,6,9-trioxaundecan-1,11-diyldi-1(4H)-pyridyl-4-yliden)bis(octylammonium)dichlorid bezeichnet werden und hat die Summenformel C₃₄H₆₀N₄O₃·2 HCl.

Ein erfindungsgemäßes Verfahren zur Herstellung der erfindungsgemäßen antimikrobiellen Verbindung umfasst nach einer ersten Ausführungsform die Schritte:
- Bereitstellen eines 4-(R¹-amino)-pyridins der Formel (II) 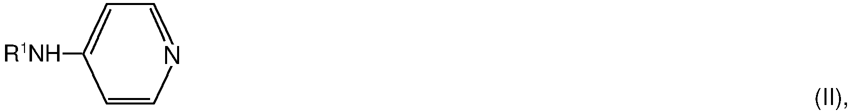 und eines disubstituierten Polyalkylenglykolderivats der Formel (III)

   X-(R²O)ₙ-R²-X (III)

   als Reaktionspartner in einem molaren Verhältnis von 2:1,
   wobei
   R¹ ein Alkylrest mit 4 bis 18 Kohlenstoffatomen oder ein Cycloalkylrest mit 5 bis 12 Kohlenstoffatomen oder ein (Poly)alkylenglykolrest mit 1 bis 6 Alkoxyeinheiten oder ein Phenylrest oder ein Benzylrest ist, und
   R² eine Alkylgruppe mit 2 oder 3 oder 4 Kohlenstoffatomen ist, und
   n eine ganze Zahl von 1 bis 15 ist, und
   X ein einwertiger anionischer Rest ist,
- Erhitzen der beiden Reaktionspartner auf eine Reaktionstemperatur in einem Bereich von 100 °C bis 180 °C für eine Reaktionsdauer von 8 bis 20 Minuten, und
- wenn die zwei einwertigen anionischen Reste X des disubstituierten Polyalkylenglykolderivats der Formel (III) den zwei einwertigen Anionen (2 A⁻) der antimikrobiellen Verbindung der Formel (I) entsprechen, Erhalten der antimikrobiellen Verbindung der Formel (I), wobei [2A] für die zwei einwertige Anionen (2 A⁻) steht,
   oder
- wenn die zwei einwertigen anionischen Reste X des disubstituierten Polyalkylenglykolderivats der Formel (III) nicht den zwei einwertigen Anionen (2 A⁻) der antimikrobiellen Verbindung der Formel (I) entsprechen, Erhalten der antimikrobiellen Verbindung der Formel (I) durch Ersetzen von jeweils zwei einwertigen anionischen Resten X in einem lonentauschverfahren durch zwei einwertige Anionen (2 A⁻) oder ein zweiwertiges Anion (A²⁻) der antimikrobiellen Verbindung der Formel (I), wobei [2A⁻] für die zwei einwertige Anionen (2A⁻) oder ein zweiwertige Anion (A²⁻) steht.

Die Reaktion der beiden Reaktionspartner verläuft innerhalb der kurzen Reaktionsdauer mit hohen Ausbeuten, sodass die Herstellung der antimikrobiellen Verbindung höchst effizient ist.

Bevorzugt kann die Reaktionstemperatur in einem Bereich von 100 °C bis 150 °C liegen und eine Reaktionsdauer von 8 bis 12 Minuten betragen. Dabei weist die erhaltene antimikrobielle Verbindung eine gute Wasserlöslichkeit auf, während die beiden Reaktionspartner wasserunlöslich sind.

Gemäß weiteren Ausführungsformen des erfindungsgemäßen Verfahrens kann das Erhitzen der beiden Reaktionspartner bevorzugt ohne zusätzliches Lösungsmittel erfolgen, wenn sich das 4-(R¹-amino)-pyridin in dem disubstituierten Polyalkylenglykolderivat löst. Ggf. kann sogar auf weitere Schritte zur Isolierung der antimikrobiellen Verbindung verzichtet werden. Alternativ kann das Erhitzen der beiden Reaktionspartner in einem polaren, vorzugsweise aprotischen Lösungsmittel erfolgen, dessen Siedepunkt höher ist als die Reaktionstemperatur. Beispiele für aprotische, polare, hochsiedende Lösungsmittel umfassen Dimethylformamid, Dimethylpropylenurea, Dimethylsulfoxid, Sulfolan.

Nach einer speziellen Ausführungsform des erfindungsgemäßen Verfahrens können zur Herstellung der antimikrobiellen Verbindung *N,N'*-(3,6,9-trioxaundecan-1,11-diyldi-1(4H)-pyridyl-4-yliden)bis(octylammonium)dichlorid als Reaktionspartner 4-(Octylamino)-pyridin und Tetraethylenglykoldichlorid (Bis-[2-(2-chlorethoxy)-ethyl]-ether) im molaren Verhältnis von 2:1 bereitgestellt werden, wobei das Erhitzen auf die Reaktionstemperatur im Bereich von 100 bis 180 °C für eine Reaktionsdauer in einem Bereich von 8 bis 20 Minuten, bevorzugt von etwa 10 Minuten und erfindungsgemäß ohne zusätzliches Lösungsmittel erfolgt.

Eine erfindungsgemäße Verwendung der antimikrobiellen Verbindung gemäß einer der vorbeschriebenen Ausführungsformen ist bei der Herstellung einer kosmetischen oder pharmazeutischen Zusammensetzung, die zur Anwendung auf Haut oder Schleimhaut vorgesehen ist und zumindest einen kosmetischen oder pharmazeutischen Basisstoff aufweist. Dabei weist die kosmetische oder pharmazeutische Zusammensetzung die antimikrobielle Verbindung in einer Konzentration von 0,001 bis 6 Gew.-% auf.

Ferner besteht eine erfindungsgemäße Verwendung der antimikrobiellen Verbindung in weiteren Ausführungsformen auch als Konservierungsmittel in technischen Zusammensetzungen innerhalb des genannten Konzentrationsbereichs, um die Haltbarkeit bzw. Lagerstabilität der technischen Zusammensetzungen zu verlängern und einen Befall durch Mikroorganismen zu verhindern. Beispiele für technische Zusammensetzungen umfassen Pflanzenschutzmittel, (Kühl-)Schmierstoffe, Lacke und Farben, und dabei insbesondere die wassermischbaren bzw. wasserbasierten Zusammensetzungen.

Die Konzentration der antimikrobiellen Verbindung in einer kosmetischen, pharmazeutischen oder technischen Zusammensetzung kann dabei in gewissem Umfang von der Länge der Alkylenglykolkette abhängig sein, durch die die beiden 4-(R¹-amino)-pyridin-Reste der erfindungsgemäßen antimikrobiellen Verbindung voneinander beabstandet sind. Längere Alkylenglykolketten sind im Vergleich mit kürzeren Alkylenglykolketten tendenziell mit höheren Konzentrationen der antimikrobiellen Verbindung verbunden, um die gleiche Wirksamkeit aufzuweisen.

Entsprechend ist eine kosmetische oder pharmazeutische Zusammensetzung, die die antimikrobielle Verbindung nach einer erfindungsgemäßen Ausführungsform und zumindest einen kosmetischen oder pharmazeutischen Basisstoff aufweist, ebenfalls Gegenstand der Erfindung. Nach einer ersten Ausführungsform weist die erfindungsgemäße kosmetische oder pharmazeutische Zusammensetzung die antimikrobielle Verbindung in einer Konzentration von 0,001 bis 6 Gew.-%, vorzugsweise 0,003 bis 3 Gew.-% auf. Entsprechend bezieht sich die Erfindung auch auf Konzentrate, die zur Herstellung einer kosmetischen oder pharmazeutischen Zusammensetzung durch Verdünnung mit einem Lösungsmittel, wie z. B. (gereinigtem) Wasser, vorgesehen sind und entsprechend höhere Konzentrationen der antimikrobiellen Verbindung aufweisen können.

Bei der kosmetischen oder pharmazeutischen Zusammensetzung handelt es sich um ein Hautpflege-, Hautbehandlungs- oder Hauterfrischungsprodukt zur Schönheitspflege oder zur Desinfektion der Haut oder Schleimhaut. Auch Haarpflegeprodukte werden dazu gezählt. In dem Bereich der Hautpflege-, Hautbehandlungs- und Hauterfrischungsprodukte sind die Übergänge zwischen kosmetischen Zusammensetzungen, die in erster Linie der Schönheitspflege dienen, und pharmazeutischen Zusammensetzungen, die einen pharmakologischen Effekt erzielen, fließend, da auch kosmetische Produkte häufig Wirkstoffe enthalten, mit denen ein pharmakologischer Effekt erzielt wird. Daher werden vorliegend kosmetische und pharmazeutische Zusammensetzungen anhand des Einsatzzwecks der erfindungsgemäßen antimikrobiellen Verbindung unterschieden: Eine kosmetische Zusammensetzung enthält die antimikrobielle Verbindung als Konservierungsstoff, d. h., dass die antimikrobielle Verbindung die kosmetische Zusammensetzung vor Verkeimung schützt. Eine pharmazeutische Zusammensetzung enthält die antimikrobielle Verbindung als antiseptischen Wirkstoff, sodass die pharmazeutische Zusammensetzung bei Anwendung auf Haut als Desinfektionsmittel antiseptisch gegen Bakterien, Pilze etc. wirksam ist.

Als kosmetischer oder pharmazeutischer Basisstoff werden alle kosmetischen und/oder pharmazeutischen Trägerstoffe und/oder Lösungsmittel verstanden, die allein oder als Gemisch die Basis für ein Hautpflege-, Hauterfrischungs- oder Hautbehandlungs- bzw. Hautdesinfektionsprodukt bilden. Die kosmetische oder pharmazeutische Zusammensetzung kann daher abhängig von der Art des Basisstoffs oder der Basisstoffe bzw. deren Anteile eine wässrige, milchige, oder halbfeste (cremige oder salbenartige) Konsistenz haben.

Nach einer weiteren Ausführungsform der erfindungsgemäßen kosmetischen oder pharmazeutischen Zusammensetzung kann der kosmetische oder pharmazeutische Basisstoff aus einer Gruppe ausgewählt sein, die gereinigtes Wasser, pflanzliche Öle und Fette und mineralische Öle und Fette aufweist. Die kosmetische oder pharmazeutische Zusammensetzung kann somit eine wässrige Zusammensetzung, d. h. mit Wasser als Basisstoff, als Lotion eine milchige Zusammensetzung (Öl-in-Wasser-Emulsion) oder als Creme oder Salbe eine halbfeste Zusammensetzung (Wasser-in-Öl/Fett-Emulsion) aufweisen, wobei ein Öl- bzw. Fettanteil einer Salbe größer ist als der einer Creme.

Je nach Einsatzzweck als Konservierungsstoff oder als Antiseptikum und ggf. auch in Abhängigkeit des jeweiligen Basisstoffs bzw. der jeweiligen Basisstoffe kann die Konzentration der antimikrobiellen Verbindung in der erfindungsgemäßen kosmetischen oder pharmazeutischen Zusammensetzung variieren:
So kann nach bestimmten Ausführungsformen der erfindungsgemäßen kosmetischen oder pharmazeutischen Zusammensetzung die Konzentration der antimikrobiellen Verbindung als Konservierungsmittel in der kosmetischen Zusammensetzung in einem Bereich von 0,001 bis 0,5 Gew.-%, beispielsweise in einem Bereich von 0,002 bis 0,1 Gew.-%, vorzugsweise von 0,003 bis 0,05 Gew.-% liegen. Bevorzugt kann die Konzentration der antimikrobiellen Verbindung als Konservierungsmittel in der kosmetischen Zusammensetzung in einem Bereich von 0,005 bis 0,03 Gew.-%, besonders bevorzugt in einem Bereich von 0,008 bis 0,02 Gew.-% liegen und insbesondere 0,0125 Gew.-% betragen.

In pharmazeutischen Zusammensetzung einer weiteren Ausführungsform der erfindungsgemäßen kosmetischen oder pharmazeutischen Zusammensetzung kann die Konzentration der antimikrobiellen Verbindung als Antiseptikum in einem Bereich von 0,005 bis 6 Gew.-%, beispielsweise in einem Bereich von 0,01 bis 3 Gew.-%, bevorzugt in einem Bereich von 0,02 bis 2 Gew.-%, besonders bevorzugt in einem Bereich von 0,03 bis 1 Gew.-% und insbesondere in einem Bereich von 0,05 bis 0,5 Gew.-% liegen.

Außerdem kann die pharmazeutische Zusammensetzung gemäß einer weiteren Ausführungsform der erfindungsgemäßen kosmetischen oder pharmazeutischen Zusammensetzung zumindest einen weiteren, mit der antimikrobiellen Verbindung kompatiblen antimikrobiellen Wirkstoff aufweisen, der aus einer Gruppe ausgewählt ist, die Phenoxyethanol, 1-Propanol und 2-Propanol, Ethanol und kationische Tenside wie Benzalkoniumchlorid aufweist.

Eine erfindungsgemäße kosmetische oder pharmazeutische Zusammensetzung kann ferner nach einer weiteren Ausführungsform zumindest einen weiteren, mit der antimikrobiellen Verbindung kompatiblen Inhaltsstoff aufweisen, der aus einer Gruppe ausgewählt ist, die Tenside, Emulgatoren, Gelbildner, Oxidationshemmer, Säure-Base-Regulatoren, Vitamine, Provitamine, Pflanzenstoffe und Pflanzenextrakte, chemische UV-Filter, mineralische UV-Filter, Farbstoffe, Duftstoffe, Proteine, Proteinhydrolysate und Geschmackstoffe aufweist.

Mit der antimikrobiellen Verbindung kompatible Wirk- bzw. Inhaltsstoffe sind beispielsweise eher kationische, amphotere oder nichtionische Wirk- oder sonstige Inhaltsstoffe. Anionische Wirk- und Inhaltsstoffe sind eher nicht mit der antimikrobiellen Verbindung kompatibel aufgrund der kationischen Struktur der antimikrobiellen Verbindung.

Weitere Ausführungsformen sowie einige der Vorteile, die mit diesen und weiteren Ausführungsformen verbunden sind, werden durch die nachfolgende ausführliche Beschreibung deutlich und besser verständlich.

Die erfindungsgemäße antimikrobielle Verbindung mit der Formel (I) lässt sich mit hoher Ausbeute effizient aus der Reaktion eines 4-(R¹-amino)-pyridins der Formel (II) mit einem disubstituierten Polyalkylenglykolderivat der Formel (III) erhalten. Zur Durchführung der Reaktion werden das 4-(R¹-amino)-pyridin der Formel (II) und das disubstituierten Polyalkylenglykolderivat der Formel (III) in einem molaren Verhältnis von 2:1 auf eine Reaktionstemperatur von 100 °C bis 180 °C für 8 bis 20 Minuten erhitzt.

Um beispielsweise als erfindungsgemäße antimikrobielle Verbindung *N*,*N*'-(3,6,9-trioxaundecan-1,11-diyldi-1(4*H*)-pyridyl-4-yliden)bis(octylammonium)dichlorid mit nachstehender Strukturformel 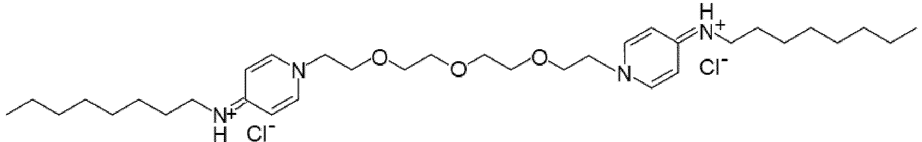 zu erhalten, werden als Reaktionspartner 4-(Octylamino)-pyridin (CAS: 64690-19-3) als 4-(R¹-amino)-pyridin der Formel (II) und Tetraethylenglykoldichlorid (auch als 1,11-Dichlor-3,6,9-trioxaundecan oder Bis-[2-(2-chlorethoxy)-ethyl]-ether bezeichnet, CAS: 638-56-2) als disubstituiertes Polyalkylenglykolderivat der Formel (III) im molaren Verhältnis von 2:1 auf die Reaktionstemperatur von 100 °C bis 150 °C ohne zusätzlichen Lösungsmittel erhitzt. Denn beim Erhitzen löst sich das kristalline 4-(Octylamino)-pyridin in Tetraethylenglykoldichlorid zu einer Reaktionslösung, die wird während der kurzen Reaktionsdauer von ca. 10 Minuten in hoher Ausbeute umgesetzt wird, was im Verlauf der Reaktion mit einer leichten Orangeverfärbung bei gleichzeitiger Viskositätserhöhung einhergeht. Die Umsetzung erfolgt nahezu vollständig, da bei Lösung des Reaktionsproduktes in Wasser keine unlöslichen Rückstände der Edukte verbleiben. Ggf. kann ein Reinigungsschritt zur vollständigen Isolation des Reaktionsprodukts erfolgen.

Alternativ kann die Reaktion auch in einem hochsiedenden, polaren Lösungsmittel durchgeführt werden, dessen Siedepunkt oberhalb der Reaktionstemperatur liegt. Anschließend kann die antimikrobielle Verbindung aus dem Lösungsmittel durch ein übliches Trennverfahren (Filtrieren, Fällen, Verdampfen etc.) isoliert werden.

Eine Vielzahl alternativer erfindungsgemäßer antimikrobieller Verbindungen entsteht durch Variation der Reaktionspartner, dem 4-(R¹-amino)-pyridin der Formel (II) und dem disubstituierten Polyalkylenglykolderivat der Formel (III).

Das 4-(R¹-amino)-pyridin der Formel (II) kann alternativ zu dem Octylrest im obigen Beispiel einen Butyl-, Pentyl-, Hexyl-, Heptyl- oder Nonyl-, Decyl-, Dodecyl-Rest usw. bis Octadecyl- bzw. Stearyl-Rest aufweisen.

Das disubstituierte Polyalkylenglykolderivat der Formel (III), das im obigen Beispiel Tetraethylenglykoldichlorid ist, kann anstelle von Chlor alternative anionische Reste, z. B. Brom, lod, oder Alkyl- oder Aryl-Sulfonyloxy-Reste aufweisen, die ggf. nach erfolgter Reaktion der beiden Reaktionspartner in einem Ionentauschverfahren ersetzt werden, um die gewünschte antimikrobielle Verbindung der Formel (I) mit den zwei einwertigen Anionen (2 A⁻) oder dem zweiwertigen Anion (A²⁻) anstelle den anionischen Resten zu erhalten. Ferner kann das disubstituierte Polyalkylenglykolderivat der Formel (III) Polyalkylenglykolketten mit abweichender Kettenlänge und/oder abweichenden Alkoxyeinheiten aufweisen.

Beispielhafte Alternativen zu Tetraethylenglykoldichlorid mit abweichender Kettenlänge umfassen 2,2'-Dichlordiethylether (bzw. Bis(2-chlorethyl)ether), Triethylenglycoldichlorid (bzw. 1,8-Dichloro-3,6-Dioxaoctan), Pentaethylenglykoldichlorid (1,14-Dichloro-3,6,9,12-tetraoxatetradecan) oder Hexaethylenglykoldichlorid (1,17- Dichloro-3,6,9,12,15-penta-oxaheptadecan) sowie deren Derivate mit von Chlor abweichenden Substituenten.

Beispielhafte Alternativen zu Tetraethylenglykoldichlorid mit abweichender Alkoxyeinheit umfassen disubstituierte Polypropylenglykolderivate, wie Tri-, Tetra-, Penta-, Hexapropylenglykoldichlorid und deren Derivate mit von Chlor abweichenden Substituenten, sowie disubstituierte Polyethylenglykol-propylenglykolderivate, die in Summe zwei bis sechs Ethylen- und Propyleneinheiten aufweisen, in zufälliger, abwechselnder oder blockweiser Reihenfolge, weshalb die möglichen Varianten aufgrund der Vielzahl der hier nicht einzeln angeführt werden, aber dennoch umfasst sein sollen. Die Propyleneinheiten können jeweils aus n-Propyl- oder i-Propyleinheiten ausgewählt werden.

Die zur Synthese der erfindungsgemäßen antimikrobiellen Verbindung verwendeten Edukte 4-(R¹-amino)-pyridin und disubstituiertes Polyalkylenglykolderivat sind beide wasserunlöslich, während die als Reaktionsprodukt erhaltene antimikrobiellen Verbindung eine gute Wasserlöslichkeit aufweist.

Versuche zur Wirksamkeit der antimikrobielle Verbindung als Konservierungsmittel wurden beispielhaft für die antimikrobielle Verbindung *N,N'-*(3,6,9-trioxaundecan-1,11-diyldi-1(4*H*)-pyridyl-4-yliden)bis(octylammonium)dichlorid durchgeführt, das aus der Reaktion von 4-(Octylamino)-pyridin und Tetraethylenglykoldichlorid im molaren Verhältnis von 2:1 erhalten wird.

Die durchgeführten Konservierungsbelastungstests erfolgten an einem Hauterfrischungsspray, einer kosmetischen Zusammensetzung auf Wasserbasis, die die antimikrobielle Verbindung als Konservierungsmittel in einer Konzentration von 0,0125 Gew.-% aufweist.

Der Testablauf erfolgte nach DIN EN ISO 11930:2019-04 durch Beimpfung von Proben des Hauterfrischungssprays mit den festgelegten Mikroorganismen (jeden Keim einzeln) und Bestimmung der Keimzahl, Lagerung der beimpften Proben unter definierten Bedingungen und Bestimmung der Keimzahlen nach festgelegten Zeitpunkten und Bestimmung der Keimzahlentwicklung. Zur Überprüfung, ob die Keimzahlen entsprechend den Vorgaben reduziert werden, werden jeweils die logarithmischen Reduktionswerte bestimmt, die zusammen mit der Keimzahlentwicklung bei 20 bei 25 °C nach Beimpfen der Proben mit den aufgeführten Keimsuspensionen in der nachfolgenden Tabelle 1 aufgeführt sind, worin alle Keimzahlen in KBE/g bzw. ml (KBE = Koloniebildende Einheiten) ausgedrückt sind:

**Tabelle 1**

| | 0 h | 7d | 14 d | 28 d |
|---|---|---|---|---|
| Pseudomonas aeruginosa ATCC 9027 | 2,0 x 10⁵ | < 1,0 x 10¹ | < 1,0 x 10¹ | < 1,0 x 10¹ |
| Logarithmische Reduktionswerte | --- | > 4,3 | > 4,3 | > 4,3 |
| Staphylococcus aureus ATCC 6538 | 2,3 x 10⁵ | < 1,0 x 10¹ | < 1,0 x 10¹ | < 1,0 x 10¹ |
| Logarithmische Reduktionswerte | --- | > 4,4 | > 4,4 | > 4,4 |
| Escherichia coli ATCC 8739 | 2,4 x 10⁵ | < 1,0 x 10¹ | < 1,0 x 10¹ | < 1,0 x 10¹ |
| Logarithmische Reduktionswerte | --- | > 4,4 | > 4,4 | > 4,4 |
| Candida albicans ATCC 10231 | 4,7 x 10⁴ | < 1,0 x 10¹ | < 1,0 x 10¹ | < 1,0 x 10¹ |
| Logarithmische Reduktionswerte | --- | > 3,7 | > 3,7 | > 3,7 |
| Aspergillus brasiliensis ATCC 16404 | 2,2 x 104 | --- | < 1,0 x 10¹ | < 1,0 x 10¹ |
| Logarithmische Reduktionswerte | --- | --- | > 3,3 | > 3,3 |

Tabelle 2 listet die für die Belastungstests definierten Bewertungskriterien A und B (logarithmische Reduktionswerte) nach DIN EN ISO 11930, wonach eine Abweichung von 0,5 log-Stufen zulässig ist.

**Tabelle 2:**

| | 0 h | 7d | 14 d | 28 d |
|---|---|---|---|---|
| Bakterien (Kriterium A) | --- | ≥ 3,0 | ≥ 3,0 * | ≥ 3,0 * |
| Bakterien (Kriterium B) | --- | --- | ≥ 3,0 | ≥ 3,0 * |
| Candida albicans (Kriterium A) | --- | ≥ 1,0 | ≥ 1,0 * | ≥ 1,0 * |
| Candida albicans (Kriterium B) | --- | --- | ≥ 1,0 | ≥ 1,0 * |
| Aspergillus brasiliensis (Kriterium A) | --- | --- | ≥ 0,0 | ≥ 1,0 * |
| Aspergillus brasiliensis (Kriterium B) | --- | --- | ≥ 0,0 | ≥ 0,0* |

| | | | | |
|---|---|---|---|---|
| * kein Anstieg seit der letzten Zählung | | | | |

Das Ergebnis zeigt, dass die kosmetische Zusammensetzung, die die antimikrobielle Verbindung als Konservierungsmittel enthält, die Anforderungen nach Kriterium A + B des Konservierungs-Belastungs-Tests nach DIN EN ISO 11930 erfüllt.

Außerdem wurden für diese kosmetische Zusammensetzung die Gesamtanzahl der aeroben mesophilen Bakterien (nach ISO 21149:2017-06 mod. bzw. Ph. Eur. 2.06.12 mod.) und die Gesamtanzahl an Hefen und Schimmelpilzen (nach ISO 16212:2017-06 mod. bzw. Ph. Eur. 2.06.12 mod.) bestimmt, die jeweils bei < 10 KBE/g (Koloniebildende Einheiten pro Gremm) lag, sodass die mikrobiologische Beschaffenheit der Proben bezüglich der durchgeführten Untersuchungen nicht zu beanstanden war.

## Patentansprüche

1. Antimikrobielle Verbindung mit der Formel (I) 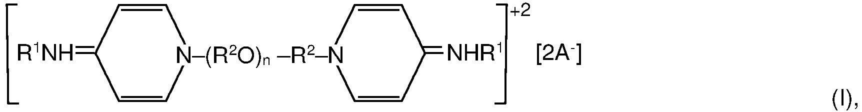 wobei
R¹ ein Alkylrest mit 4 bis 18 Kohlenstoffatomen oder ein Cycloalkylrest mit 5 bis 12 Kohlenstoffatomen oder ein (Poly)alkylenglykolrest mit 1 bis 6 Alkoxyeinheiten oder ein Phenylrest oder ein Benzylrest ist, und
R² eine Alkylgruppe mit 2 oder 3 oder 4 Kohlenstoffatomen ist, und
n eine ganze Zahl von 1 bis 15 ist, und
[2A⁻] für zwei einwertige Anionen (2 A⁻) oder ein zweiwertiges Anion (A²⁻) steht.

2. Antimikrobielle Verbindung nach Anspruch 1
aus der Reaktion eines 4-(R¹-amino)-pyridins der Formel (II) 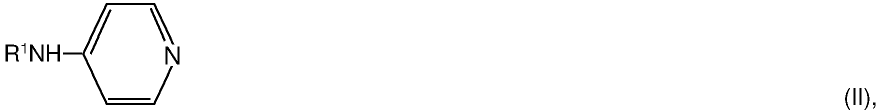
mit einem disubstituierten Polyalkylenglykolderivat der Formel (III)
X-(R²O)ₙ-R²-X (III)
in einem molaren Verhältnis von 2:1,
wobei
R¹ ein Alkylrest mit 4 bis 18 Kohlenstoffatomen oder ein Cycloalkylrest mit 5 bis 12 Kohlenstoffatomen oder ein (Poly)alkylenglykolrest mit 1 bis 6 Alkoxyeinheiten oder ein Phenylrest oder ein Benzylrest ist, und
R² eine Alkylgruppe mit 2 oder 3 oder 4 Kohlenstoffatomen ist, und
n eine ganze Zahl von 1 bis 15 ist, und
X ein einwertiger anionischer Rest ist, wobei
entweder die zwei einwertigen anionischen Reste X des disubstituierten Polyalkylenglykolderivats der Formel (III) den zwei einwertigen Anionen (2 A⁻) der antimikrobiellen Verbindung der Formel (I) entsprechen,
oder nach erfolgter Reaktion des 4-(R¹-amino)-pyridins der Formel (II) mit dem disubstituierten Polyalkylenglykolderivat der Formel (III) die zwei einwertigen anionischen Reste X in einem lonentauschverfahren durch die zwei einwertigen Anionen (2 A⁻) oder das zweiwertige Anion (A²⁻) der antimikrobiellen Verbindung der Formel (I) ersetzt werden.

3. Antimikrobielle Verbindung nach Anspruch 1 oder 2, wobei die zwei einwertigen Anionen (2 A) aus einer Gruppe ausgewählt ist, die Chlorid, Bromid, Fluorid, Iodid, Nitrat, Acetat aufweist, wobei Chlorid und Bromid bevorzugt sind.

4. Antimikrobielle Verbindung nach Anspruch 1 oder 2, wobei das zweiwertige Anion (A²⁻) aus einer Gruppe ausgewählt ist, die Sulfat, Oxalat aufweist.

5. Antimikrobielle Verbindung nach zumindest einem der Ansprüche 2 bis 4, wobei der einwertige anionische Rest X aus einer Gruppe ausgewählt ist, die Chlorid, Bromid, Iodid, Alkyl-Sulfonyloxyreste, Aryl-Sulfonyloxyreste aufweist.

6. Antimikrobielle Verbindung nach zumindest einem der Ansprüche 2 bis 5, wobei die antimikrobiellen Verbindung N,N'-(3,6,9-trioxaundecan-1,11-diyldi-1(4H)-pyridyl-4-yliden)bis(octylammonium)dichlorid ist, bei der in der Formel (I)
R¹ ein linearer Octylrest ist, und
R² eine Ethylgruppe ist, und
n gleich 3 ist, und
die zwei einwertigen Anionen (2 A⁻) Chlorid sind.

7. Verfahren zur Herstellung der antimikrobiellen Verbindung nach zumindest einem der Ansprüche 1 bis 6,
**umfassend die Schritte**
- Bereitstellen eines 4-(R¹-amino)-pyridins der Formel (II) 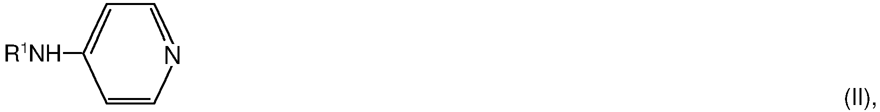
und eines disubstituierten Polyalkylenglykolderivat der Formel (III)
X-(R²O)ₙ-R²-X (III)
als Reaktionspartner in einem molaren Verhältnis von 2:1,
wobei
R¹ ein Alkylrest mit 4 bis 18 Kohlenstoffatomen oder ein Cycloalkylrest mit 5 bis 12 Kohlenstoffatomen oder ein (Poly)alkylenglykolrest mit 1 bis 6 Alkoxyeinheiten oder ein Phenylrest oder ein Benzylrest ist, und
R² eine Alkylgruppe mit 2 oder 3 oder 4 Kohlenstoffatomen ist, und
n eine ganze Zahl von 1 bis 15 ist, und
X ein einwertiger anionischer Rest ist,
- Erhitzen der beiden Reaktionspartner auf eine Reaktionstemperatur in einem Bereich von 100 °C bis 180 °C für eine Reaktionsdauer in einem Bereich von 8 bis 20 Minuten und,
- wenn die zwei einwertigen anionischen Reste X des disubstituierten Polyalkylenglykolderivats der Formel (III) den zwei einwertigen Anionen (2 A⁻) der antimikrobiellen Verbindung der Formel (I) entsprechen, Erhalten der antimikrobiellen Verbindung der Formel (I), wobei [2A⁻] für die zwei einwertige Anionen (2 A⁻) steht,
oder
- wenn die zwei einwertigen anionischen Reste X des disubstituierten Polyalkylenglykolderivats der Formel (III) nicht den zwei einwertigen Anionen (2 A⁻) der antimikrobiellen Verbindung der Formel (I) entsprechen, Erhalten der antimikrobiellen Verbindung der Formel (I) durch Ersetzen von jeweils zwei einwertigen anionischen Resten X in einem lonentauschverfahren durch zwei einwertige Anionen (2 A⁻) oder ein zweiwertiges Anion (A²⁻) der antimikrobiellen Verbindung der Formel (I), wobei [2A] für die zwei einwertige Anionen (2A) oder ein zweiwertiges Anion (A²⁻) steht.

8. Verfahren nach Anspruch 7, wobei das Erhitzen der beiden Reaktionspartner
- ohne Lösungsmittel erfolgt,
oder
- in einem polaren Lösungsmittel erfolgt, dessen Siedepunkt höher ist als die Reaktionstemperatur, wobei das polare Lösungsmittel bevorzugt ein aprotisches Lösungsmittel ist.

9. Verfahren nach Anspruch 7 oder 8, wobei die Reaktionspartner 4-(Octylamino)-pyridin und Tetraethylenglykoldichlorid im molaren Verhältnis von 2:1 sind, wobei das Erhitzen auf die Reaktionstemperatur im Bereich von 100 °C bis 180 °C für eine Reaktionsdauer in einem Bereich von 8 bis 20 Minuten ohne zusätzliches Lösungsmittel erfolgt.

10. Verwendung der antimikrobiellen Verbindung nach zumindest einem der Ansprüche 1 bis 6 zur Herstellung einer kosmetischen oder pharmazeutischen Zusammensetzung zur Anwendung auf Haut, wobei die kosmetische oder pharmazeutische Zusammensetzung zumindest einen kosmetischen oder pharmazeutischen Basisstoff und die antimikrobielle Verbindung in einer Konzentration von 0,001 bis 6 Gew.% aufweist.

11. Kosmetische oder pharmazeutische Zusammensetzung, geeignet zur Anwendung auf Haut, die die antimikrobielle Verbindung nach zumindest einem der Ansprüche 1 bis 6 und zumindest einen kosmetischen oder pharmazeutischen Basisstoff aufweist, wobei die kosmetische oder pharmazeutische Zusammensetzung die antimikrobielle Verbindung in einer Konzentration von 0,001 bis 6 Gew.-%, vorzugsweise von 0,003 bis 3 Gew.-%, aufweist.

12. Kosmetische oder pharmazeutische Zusammensetzung nach Anspruch 11, wobei der kosmetische oder pharmazeutische Basisstoff aus einer Gruppe ausgewählt ist, die gereinigtes Wasser, pflanzliche Öle und Fette und mineralische Öle und Fette aufweist.

13. Kosmetische oder pharmazeutische Zusammensetzung nach Anspruch 11 oder 12, wobei die Konzentration der antimikrobiellen Verbindung als Konservierungsmittel in der kosmetischen Zusammensetzung in einem Bereich von 0,001 bis 0,5 Gew.-%, vorzugsweise von 0,003 bis 0,05 Gew.-%, besonders bevorzugt in einem Bereich von 0,005 bis 0,03 Gew.-%, insbesondere in einem Bereich von 0,008 bis 0,02 Gew.-% liegt.

14. Kosmetische oder pharmazeutische Zusammensetzung nach Anspruch 11 oder 12, wobei die Konzentration der antimikrobiellen Verbindung als Antiseptikum in der pharmazeutische Zusammensetzung in einem Bereich von 0,005 bis 6 Gew.-%, vorzugsweise von 0,01 bis 3 Gew.-%, besonders bevorzugt in einem Bereich von 0,02 bis 2 Gew.-%, und insbesondere in einem Bereich von 0,03 bis 1 Gew.-%, liegt, und/oder
die pharmazeutische Zusammensetzung zumindest einen weiteren, mit der antimikrobiellen Verbindung kompatiblen antimikrobiellen Wirkstoff aufweist, der aus einer Gruppe ausgewählt ist, die Phenoxyethanol, 1-Propanol und 2-Propanol, Ethanol und kationische Tenside wie Benzalkoniumchlorid aufweist.

15. Kosmetische oder pharmazeutische Zusammensetzung nach zumindest einem der Ansprüche 11 bis 14, wobei die kosmetische oder pharmazeutische Zusammensetzung zumindest einen weiteren, mit der antimikrobiellen Verbindung kompatiblen Inhaltsstoff aufweist, der aus einer Gruppe ausgewählt ist, die Tenside, Emulgatoren, Gelbildner, Oxidationshemmer, Säure-Base-Regulatoren, Vitamine, Provitamine, Pflanzenstoffe und Pflanzenextrakte, chemische UV-Filter, mineralische UV-Filter, Farbstoffe, Duftstoffe, Proteine, Proteinhydrolysate und Geschmackstoffe aufweist.

## Claims

1. An antimicrobial compound with the formula (I) 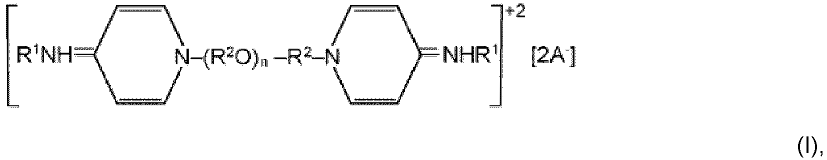 wherein
R¹ is an alkyl radical with 4 to 18 carbon atoms or a cycloalkyl radical with 5 to 12 carbon atoms or a (poly)alkylene glycol radical with 1 to 6 alkoxy units or a phenyl radical or a benzyl radical, and
R² is an alkyl group with 2 or 3 or 4 carbon atoms, and
n is an integer from 1 to 15, and
[2A⁻] represents two monovalent anions (2 A⁻) or one divalent anion (A²⁻).

2. The antimicrobial compound according to claim 1
from the reaction of a 4-(R¹-amino)-pyridine of the formula (II) 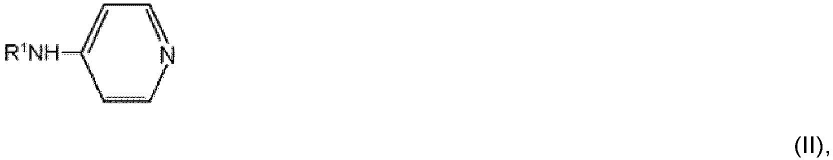
with a disubstituted polyalkylene glycol derivative of the formula (III)
X-(R²O)ₙ-R²-X (III)
at a molar ratio of 2:1,
wherein
R¹ is an alkyl radical with 4 to 18 carbon atoms or a cycloalkyl radical with 5 to 12 carbon atoms or a (poly)alkylene glycol radical with 1 to 6 alkoxy units or a phenyl radical or a benzyl radical, and
R² is an alkyl group with 2 or 3 or 4 carbon atoms, and
n is an integer from 1 to 15, and
X is a monovalent anionic radical, wherein
either the two monovalent anionic radicals X of the disubstituted polyalkylene glycol derivative of the formula (III) correspond to the two monovalent anions (2 A⁻) of the antimicrobial compound of the formula (I),
or the two monovalent anionic radicals X are replaced in an ion exchange process with the two monovalent anions (2 A⁻) or the bivalent anion (A²⁻) of the antimicrobial compound of the formula (I) after reaction of the 4-(R¹-amino)-pyridine of the formula (II) with the disubstituted polyalkylene glycol derivative of the formula (III) has taken place.

3. The antimicrobial compound according to claim 1 or 2, wherein the two monovalent anions (2 A⁻) is selected from a group, which has chloride, bromide, fluoride, iodide, nitrate, acetate, wherein chloride and bromide are preferred.

4. The antimicrobial compound according to claim 1 or 2, wherein the bivalent anion (A²⁻) is selected from a group, which has sulfate, oxalate.

5. The antimicrobial compound according to at least any one of claims 2 to 4, wherein the monovalent anionic radical X is selected from a group, which has chloride, bromide, iodide, alkyl-sulfonyloxy and aryl-sulfonyloxy radicals.

6. The antimicrobial compound according to at least any one of claims 2 to 5, wherein the antimicrobial compound is N,N'- (3,6,9-trioxaundecane-1,11-diyldi-1(4H)-pyridyl-4-yliden)bis(octylammonium)dichloride, in the case of which
R¹ is a linear octyl radical, and
R² is an ethyl group, and
n equals 3, and
the two monovalent anions (2 A⁻) are chloride in the formula (I).

7. A process for preparing the antimicrobial compound according at least any one of claims 1 to 6,
**comprising the steps of:**
- providing a 4-(R¹-amino)-pyridine of the formula (II) 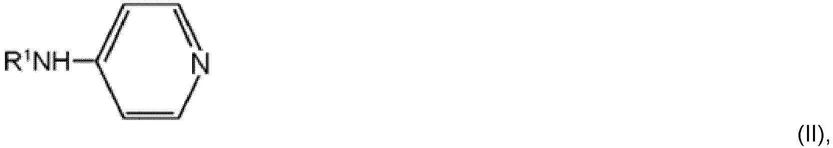
and a disubstituted polyalkylene glycol derivative of the formula (III)
X-(R²O)ₙ-R²-X (III),
as reaction partner in a molar ratio of 2:1,
wherein
R¹ is an alkyl radical with 4 to 18 carbon atoms or a cycloalkyl radical with 5 to 12 carbon atoms or a (poly)alkylene glycol radical with 1 to 6 alkoxy units or a phenyl radical or a benzyl radical, and
R² is an alkyl group with 2 or 3 or 4 carbon atoms, and
n is an integer from 1 to 15, and
X is a monovalent anionic radical,
- heating the two reaction partners to a reaction temperature in a range of 100 °C to 180 °C for a reaction time in a range of 8 to 20 minutes, and
- when the two monovalent anionic radicals X of the disubstituted polyalkylene glycol derivative of the formula (III) correspond to the two monovalent anions (2 A⁻) of the antimicrobial compound of the formula (I), obtaining the antimicrobial compound of the formula (I),
wherein [2A⁻] represents the two monovalent anions (2 A⁻),
or
- when the two monovalent anionic radicals X of the disubstituted polyalkylene glycol derivative of the formula (III) do not correspond to the two monovalent anions (2 A⁻) of the antimicrobial compound of the formula (I), obtaining the antimicrobial compound of the formula (I) by replacing two respective monovalent anionic radicals X in an ion exchange process with two monovalent anions (2 A⁻) or a bivalent anion (A²⁻) of the antimicrobial compound of the formula (I), wherein [2A⁻] represents the two monovalent anions (2A⁻) or a bivalent anion (A²⁻).

8. The process according to claim 7, wherein the heating of the two reaction partners
- takes place without solvent or
- takes place in a polar solvent, the boiling point of which is higher than the reaction temperature, wherein the polar solvent is preferably an aprotic solvent.

9. The process according to claim 7 or 8, wherein the reaction partners are 4-(octylamino)-pyridine and tetraethylene glycol dichloride in the molar ratio of 2:1, wherein the heating to the reaction temperature takes place in the range of 100 °C to 180 °C for a reaction time in a range of 8 to 20 minutes without additional solvent.

10. Use of the antimicrobial compound according to at least any one of claims 1 to 6 for preparing a cosmetic or pharmaceutical composition for the application to skin, wherein the cosmetic or pharmaceutical composition has at least one cosmetic or pharmaceutical base substance and the antimicrobial compound in a concentration of 0.001 to 6% by weight.

11. A cosmetic or pharmaceutical composition, suitable for the application to skin, which has the antimicrobial compound according to at least any one of claims 1 to 6 and at least one cosmetic or pharmaceutical base substance, wherein the cosmetic or pharmaceutical composition has the antimicrobial compound in a concentration of 0.001 to 6% by weight, preferably of 0.003 to 3% by weight.

12. The cosmetic or pharmaceutical composition according to claim 11, wherein the cosmetic or pharmaceutical base substance is selected from a group, which has purified water, vegetable oils and fats and mineral oils and fats.

13. The cosmetic or pharmaceutical composition according to claim 11 or 12, wherein the concentration of the antimicrobial compound as preservative in the cosmetic composition lies in a range of 0.001 to 0.5% by weight, preferably from 0.003 to 0.05% by weight, particularly preferably in a range of 0.005 to 0.03% by weight, in particular in a range of 0.008 to 0.02% by weight.

14. The cosmetic or pharmaceutical composition according to claim 11 or 12, wherein the concentration of the antimicrobial compound as antiseptic in the pharmaceutical composition lies in a range of 0.005 to 6% by weight, preferably of 0.01 to 3% by weight, particularly preferably in a range of 0.02 to 2% by weight, and in particular in a range of 0.03 to 1% by weight, and/or
the pharmaceutical composition has at least one further antimicrobial active ingredient, which is compatible with the antimicrobial compound and which is selected from a group, which has phenoxyethanol, 1-propanol and 2-propanol, ethanol und cationic surfactants, such as benzalkonium chloride.

15. The cosmetic or pharmaceutical composition according to at least any one of claims 11 to 14, wherein the cosmetic or pharmaceutical composition has at least one further ingredient, which is compatible with the antimicrobial compound and which is selected from a group, which has surfactants, emulsifiers, gelling agents, oxidation inhibitors, acid-base regulators, vitamins, provitamins, plant substances and plant extracts, chemical UV filters, mineral UV filters, colors, fragrances, proteins, protein hydrolysates and flavoring substances.

## Revendications

1. Composé antimicrobien de formule (I), 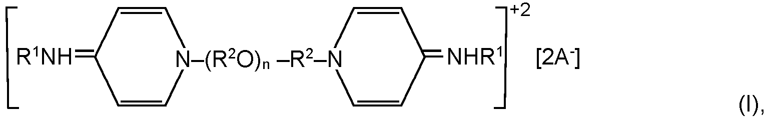
R¹ étant un groupe alkyle ayant 4 à 18 atomes de carbone ou un groupe cycloalkyle ayant 5 à 12 atomes de carbone ou un groupe (poly)alkylèneglycol ayant 1 à 6 unités alcoxy ou un groupe phényle ou un groupe benzyle, et
R² étant un groupe alkyle ayant 2 ou 3 ou 4 atomes de carbone, et
n étant un nombre entier de 1 à 15, et,
[2A⁻] représentant deux anions monovalents (2 A⁻) ou un anion divalent (A²⁻).

2. Composé antimicrobien selon la revendication 1
résultant de la réaction d'une 4-(R¹-amino)-pyridine de formule (II), 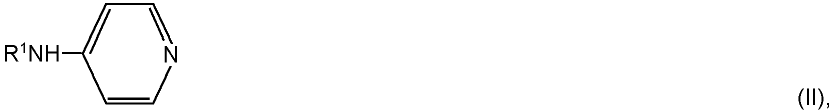
avec un dérivé de polyalkylène glycol disubstitué de formule (III)
X-(R²O)ₙ-R²-X (III)
dans un rapport molaire de 2:1,
R¹ étant un groupe alkyle ayant 4 à 18 atomes de carbone ou un groupe cycloalkyle ayant 5 à 12 atomes de carbone ou un groupe (poly)alkylèneglycol ayant 1 à 6 unités alcoxy ou un groupe phényle ou un groupe benzyle, et
R² étant un groupe alkyle ayant 2 ou 3 ou 4 atomes de carbone, et
n étant un nombre entier de 1 à 15, et
X étant un radical anionique monovalent,
soit les deux radicaux anioniques monovalents X du dérivé de polyalkylène glycol disubstitué de formule (III) correspondant aux deux anions monovalents (2A⁻) du composé antimicrobien de formule (I),
soit, après la réaction de la 4-(R¹-amino)-pyridine de formule (II) avec le dérivé de polyalkylène glycol disubstitué de formule (III), les deux radicaux anioniques monovalents X étant remplacés dans un procédé d'échange d'ions par les deux anions monovalents (2 A⁻) ou l'anion bivalent (A²⁻) du composé antimicrobien de formule (I).

3. Composé antimicrobien selon la revendication 1 ou 2, dans lequel les deux anions monovalents (2 A⁻) est choisi dans un groupe comprenant le chlorure, le bromure, le fluorure, l'iodure, le nitrate, l'acétate, étant entendu que le chlorure et le bromure sont à privilégier.

4. Composé antimicrobien selon la revendication 1 ou 2, dans lequel l'anion divalent (A²⁻) est choisi dans un groupe comprenant le sulfate, l'oxalate.

5. Composé antimicrobien selon au moins l'une des revendications 2 à 4, dans lequel le radical anionique monovalent X est choisi dans un groupe comprenant le chlorure, le bromure, l'iodure, des radicaux alkyl-sulfonyloxy, des radicaux aryl-sulfonyloxy.

6. Composé antimicrobien selon au moins l'une des revendications 2 à 5, dans lequel le composé antimicrobien est le dichlorure de N,N'-(3,6,9-trioxaundécane-1,11-diyldi-1(4H)-pyridyl-4-ylidène)bis(octylammonium), étant entendu que, dans la formule (I),
R¹ est un groupe octyle linéaire, et
R² est un groupe éthyle, et
n est égal à 3, et
les deux anions monovalents (2 A⁻) sont le chlorure.

7. Procédé de préparation du composé antimicrobien selon au moins l'une des revendications 1 à 6,
**comprenant les étapes** :
- préparation d'une 4-(R¹-amino)-pyridine de formule (II) 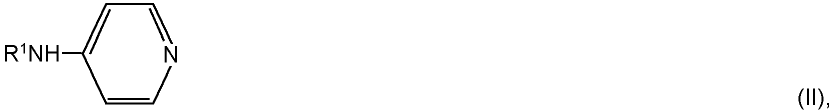
et d'un dérivé de polyalkylène glycol disubstitué de formule (III)
X-(R²O)ₙ-R²-X (III)
comme réactif dans un rapport molaire de 2:1,
R¹ étant un groupe alkyle ayant 4 à 18 atomes de carbone ou un groupe cycloalkyle ayant 5 à 12 atomes de carbone ou un groupe (poly)alkylèneglycol ayant 1 à 6 unités alcoxy ou un groupe phényle ou un groupe benzyle, et
R² étant un groupe alkyle ayant 2 ou 3 ou 4 atomes de carbone, et
n étant un nombre entier de 1 à 15, et
X étant un groupe anionique monovalent,
- chauffage des deux réactifs à une température de réaction comprise dans une plage de 100 à 180 °C pendant une durée de réaction comprise dans une plage de 8 à 20 minutes, et
- lorsque les deux radicaux anioniques monovalents X du dérivé de polyalkylèneglycol disubstitué de formule (III) correspondent aux deux anions monovalents (2 A⁻) du composé antimicrobien de formule (I), l'obtention du composé antimicrobien de formule (I), [2A⁻] représentant les deux anions monovalents (2 A⁻), ou
- lorsque les deux radicaux anioniques monovalents X du dérivé de polyalkylèneglycol disubstitué de formule (III) ne correspondent pas aux deux anions monovalents (2 A⁻) du composé antimicrobien de formule (I), l'obtention du composé antimicrobien de formule (I) en remplaçant chaque fois deux radicaux anioniques monovalents X dans un procédé d'échange d'ions par deux anions monovalents (2 A⁻) ou un anion divalent (A²⁻) du composé antimicrobien de formule (I), [2A⁻] représentant les deux anions monovalents (2A⁻) ou un anion divalent (A²⁻).

8. Procédé selon la revendication 7, dans lequel le chauffage des deux réactifs est effectué
- sans solvant,
ou
- dans un solvant polaire dont le point d'ébullition est supérieur à la température de réaction, le solvant polaire étant de préférence un solvant aprotique.

9. Procédé selon la revendication 7 ou 8, dans lequel les réactifs sont la 4-(octylamino)-pyridine et le dichlorure de tétraéthylèneglycol dans un rapport molaire de 2:1, le chauffage à la température de réaction dans la plage de 100 à 180°C pendant une durée de réaction dans une plage de 8 à 20 minutes étant effectué sans solvant supplémentaire.

10. Utilisation du composé antimicrobien selon au moins l'une des revendications 1 à 6 pour la préparation d'une composition cosmétique ou pharmaceutique destinée à être appliquée sur la peau, la composition cosmétique ou pharmaceutique comprenant au moins une base cosmétique ou pharmaceutique et le composé antimicrobien à une concentration de 0,001 à 6 % en poids.

11. Composition cosmétique ou pharmaceutique, convenant à une application sur la peau, comprenant le composé antimicrobien selon au moins l'une des revendications 1 à 6 et au moins une base cosmétique ou pharmaceutique, la composition cosmétique ou pharmaceutique comprenant le composé antimicrobien à une concentration de 0,001 à 6 % en poids, préférablement de 0,003 à 3 % en poids.

12. Composition cosmétique ou pharmaceutique selon la revendication 11, dans laquelle la base cosmétique ou pharmaceutique est choisie dans un groupe comprenant l'eau purifiée, des huiles et graisses végétales et des huiles et graisses minérales.

13. Composition cosmétique ou pharmaceutique selon la revendication 11 ou 12, dans laquelle la concentration du composé antimicrobien en tant que conservateur dans la composition cosmétique se situe dans une plage de 0,001 à 0,5 % en poids, préférablement de 0,003 à 0,05 % en poids, plus préférablement dans une plage de 0,005 à 0,03 % en poids, en particulier dans une plage de 0,008 à 0,02 % en poids.

14. Composition cosmétique ou pharmaceutique selon la revendication 11 ou 12, dans laquelle la concentration du composé antimicrobien en tant qu'antiseptique dans la composition pharmaceutique se situe dans une plage de 0,005 à 6 % en poids, préférablement de 0,01 à 3 % en poids, plus préférablement dans une plage de 0,02 à 2 % en poids, et en particulier dans une plage de 0,03 à 1 % en poids, et/ou la composition pharmaceutique comprend au moins un autre agent antimicrobien compatible avec le composé antimicrobien, choisi dans un groupe comprenant le phénoxyéthanol, le 1-propanol et le 2-propanol, l'éthanol et des agents tensioactifs cationiques tels que le chlorure de benzalkonium.

15. Composition cosmétique ou pharmaceutique selon au moins l'une des revendications 11 à 14, la composition cosmétique ou pharmaceutique comprenant au moins un autre ingrédient compatible avec le composé antimicrobien, choisi dans un groupe, comprenant des tensioactifs, des émulsifiants, des gélifiants, des inhibiteurs d'oxydation, des régulateurs acido-basiques, des vitamines, des provitamines, des substances végétales et des extraits végétaux, des filtres UV chimiques, des filtres UV minéraux, des colorants, des parfums, des protéines, des hydrolysats de protéines et des agents de sapidité.
